# EUROPEAN PATENT SPECIFICATION

(11) **EP 3 443 898 B1**
(45) Date of publication and mention of the grant of the patent: **18.12.2019**
(21) Application number: 17186739.3
(22) Date of filing: 17.08.2017
(51) Int. Cl.: A61B 5/05, A61B 5/06, A61B 5/00, A61B 90/17, A61B 90/00

(54) **A MEDICAL IMAGING SYSTEM**
SYSTEM ZUR MEDIZINISCHEN BILDGEBUNG
SYSTÈME D'IMAGERIE MÉDICALE

(43) Date of publication of application: 20.02.2019
(73) Proprietor: Micrima Limited, Bristol BS2 0EL (GB)
(72) Inventor: BANNISTER, Peter, Bristol, BS2 0EL (GB); FARLEY, Andrew, Bristol, BS2 0EL (GB)
(74) Representative: Haseltine Lake Kempner LLP

(56) References cited:
- WO-A1-2006/028395
- GB-A- 2 540 995
- US-A- 5 569 266
- US-A1- 2005 096 589
- US-A1- 2011 313 288
- US-A1- 2014 235 962
- US-A1- 2014 276 031
- OLOUMI D ET AL: "Tracking a biopsy needle inside a breast using UWB circular-SAR", 2015 IEEE INTERNATIONAL SYMPOSIUM ON ANTENNAS AND PROPAGATION & USNC/URSI NATIONAL RADIO SCIENCE MEETING. PROCEEDINGS IEEE PISCATAWAY, NJ, USA, 2015, pages 534-535, XP002777767, ISBN: 978-1-4799-7815-1

## Description

The invention relates to a medical imaging system and particularly, although not exclusively, to an apparatus which incorporates guidance of an implantable marker to a target.

Various medical imaging techniques are known for examining the human body. Such imaging techniques may be used to interrogate tissues and organs in order to identify abnormalities, such as tumours or lesions. For example, X-ray (mammography), microwave imaging, ultrasound, MRI are all common imaging modalities. Such techniques are commonly used to examine breast tissue, but may also be used in other areas of the body, such as the liver, pancreas, prostate, thyroid, lungs, ovaries and lymph nodes.

Once identified, a target, such as a tumour, can be treated using a suitable procedure, such as surgical resection.

Precise and accurate localization of the target is critical in order to ensure complete removal of the tumour with adequate margins. If the tumour is not completely excised, patients need to undergo a further operation to remove any remaining cancerous tissue. Precise and accurate localization also helps to avoid excision of excess breast tissue which could result in adverse cosmetic results.

Techniques such as wire-guided localization (WGL), Radioguided Occult Lesion Localization (ROLL) and Radio Seed Localization (RSL), can be used to provide a marker which assists the surgeon in localizing the lesion. However, these techniques rely on the target being accurately relocated after the initial identification.

Known systems for monitoring the position of a marker delivery device within the human body are described in US 2005/0096589, US 2011/0313288 and Oloumi D et al, Tracking a Biopsy Needle Inside a Breast Using UWB Circular-SAR, 2015, pages 534-535, ISBN: 978-1-4799-7815-1.

The invention seeks to provide a system which improves the accuracy of the placement of a marker at the site of a target.

In accordance with an aspect of the invention, there is provided a medical imaging system comprising: a microwave antenna array comprising a transmitting antenna and a plurality of receiving antennae, wherein the transmitting antenna is configured to transmit microwave signals so as to illuminate a body part of a patient and the receiving antennae are configured to receive the microwave signals following scattering within the body part; a processor configured to process the scattered microwave signals and generate an output indicative of the internal structure of the body part to identify a target within the body part; and a delivery device comprising a delivery needle for implanting a marker at the site of the target, the delivery device being movable relative to the microwave antenna array. The delivery needle has an integrated coaxial feed line for supplying microwave signals to the tip of the needle, such that the delivery needle itself is configured to radiate microwave signals. The receiving antennae are further configured to receive microwave signals radiated by the delivery needle and the processor is further configured to use the received microwave signals radiated by the delivery needle to monitor a position of the delivery needle as it is guided to the target within the body part.

The processor may be further configured to guide the delivery needle to the target within the body part.

The microwave antenna array may be formed on a substrate. The substrate may comprise one or more openings configured to receive the delivery needle to provide access to the body part.

The openings may be conical.

The openings may be provided with sealing gaskets.

The openings may comprise one or more slots.

The substrate may comprise a plurality of said openings and the processor may be configured to select one of the plurality of openings for introducing the delivery needle.

The delivery device may be mounted on an articulated arm configured to maneuver the delivery device relative to the antenna array.

The articulated arm may be a robotic arm.

The marker may be a microwave marker which is provided at the tip of the delivery needle and the processor may be configured to monitor the position of the delivery needle using the marker as it is guided to the target within the body part before being implanted.

The processor may be configured to perform a first data acquisition and analysis operation when identifying the target and to perform a second data acquisition and analysis operation when guiding the delivery needle, the second data acquisition and analysis operation being faster than the first data acquisition and analysis operation.

The system may further comprise a marker detector which is configured to detect the marker after it is implanted at the site of the target in order to locate the target during a surgical procedure.

The marker may be a magnetic marker.

The marker may be an electromagnetic marker, such as microwave marker.

The marker may be biodegradable.

The delivery needle may be configured to extract a biopsy from the target.

In accordance with an arrangement not falling within the scope of the claims, there is provided a medical imaging method comprising: illuminating a body part of a patient with microwave signals emitted by a transmitting antenna of an microwave antenna array; receiving the microwave signals following scattering within the body part at a plurality of receiving antennae of the microwave antenna array; processing the scattered microwave signals to generate an output indicative of the internal structure of the body part; identifying a target within the body part from the output; guiding a delivery needle to the target within the body part by monitoring microwave signals scattered or emitted by the delivery needle using the receiving antennae; and implanting a marker at the site of the target via the delivery needle.

The method may further comprise extracting a biopsy from the target.

The biopsy may be extracted from the target using the delivery needle prior to the marker being implanted.

The delivery needle may not be removed from the body part between extracting the biopsy and implanting the marker.

The method may further comprise locating the target using the marker during a subsequent surgical procedure so as to guide a surgical device to the target.

The marker may be located using a marker detector.

For a better understanding of the invention, and to show more clearly how it may be carried into effect, reference will now be made, by way of example, to the accompanying drawings, in which:-
Figure 1 is a system diagram of a medical imaging system according to an embodiment of the invention;
Figure 2 is a schematic side view of an example implementation of the medical imaging system;
Figure 3 is a schematic view of imaging portion of the medical imaging system;
Figure 4 is a flowchart depicting a sampling method;
Figure 5 is perspective cross-sectional view of an antenna shell of the medical imaging system;
Figure 6 is a perspective view of an alternative antenna shell used in the medical imaging system; and
Figure 7 is an exploded view of an assembly comprising the antenna shell of Figure 6.

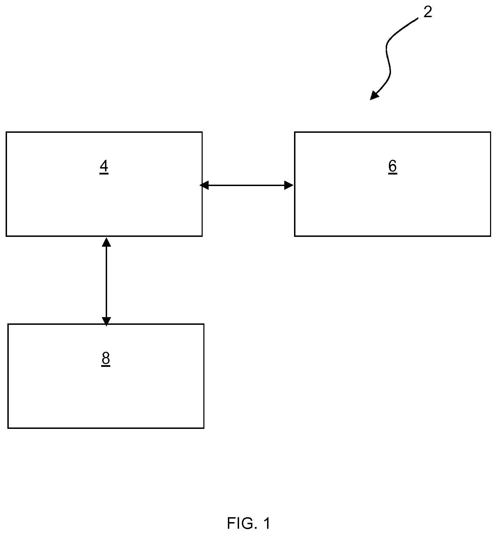

Figure 1 shows a medical imaging system 2 according to an embodiment of the invention. The medical imaging system generally comprises a processor 4, a microwave antenna array 6 in communication with the processor 4, and a delivery device 8 in communication with the processor 4.

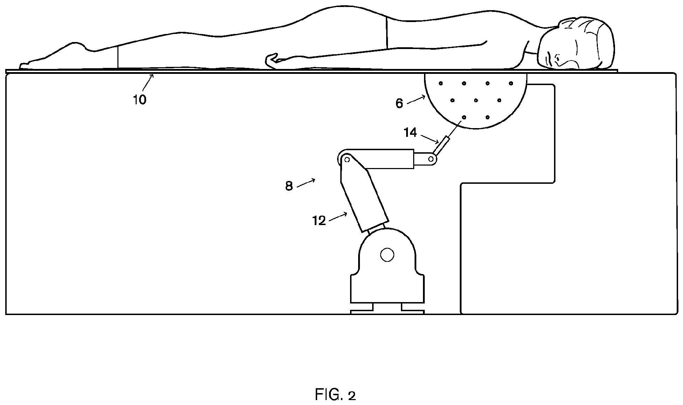

As shown in Figure 2, the microwave antenna array 6 may form part of a table 10 on which a patient lies in a prone position. The delivery device 8 is connected to the table 10 or otherwise located in a fixed or known position relative to the table 10. The delivery device 8 comprises an articulated arm 12 which carries a delivery needle 14. The articulated arm 12 allows the delivery needle 14 to be maneuvered relative to the antenna array 6.

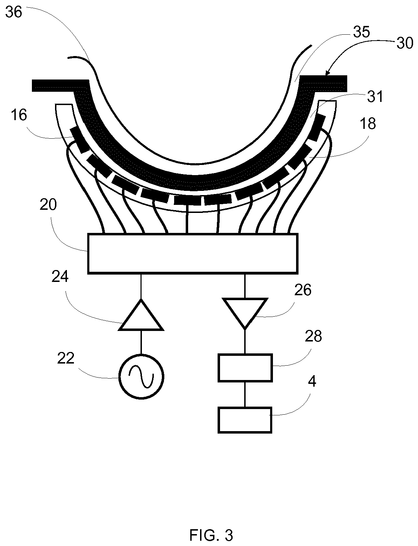

As shown in Figure 3, the antenna array 6 comprises a plurality N of antennae 16 which are arranged over the surface of, or within, a shell substrate 18. The shell 18 has a curved profile as shown. In particular, the shell 18 is part or hemi-spherical and is configured to approximate the shape of a breast. The antennae 16 are arranged over the shell 18 such that they all point to a common focal point.

The antennae 16 are each electrically connected to a switching matrix 20. The switching matrix 20 is in turn connected to both a transmit path and a receive path. The transmit path comprises a signal generator 22 coupled to an amplifier 24. The receive path comprises an amplifier 26 coupled to a detector 28 and the processor 4.

The switching matrix 20 selectively couples each of the antennae 16 to either the transmit path or the receive path.

The antenna array 6 is operated in a multi-static fashion. Specifically, the switching matrix 20 is controlled so as to connect one of the antennae 16 to the transmit path and the remaining antennae 16 to the receive path. The signal generator 22 generates a stepped frequency continuous wave (CW) signal which is amplified by the amplifier 24 and then transmitted by the antenna 16 connected to the transmit path. The stepped frequency continuous wave signal is a sequential series of pulses of continuous wave energy, where each pulse has its frequency stepped up across a range of frequencies, typically within the 3-8 GHz range. The other antennae 16 receive the transmitted signal and the received signal is detected and then recorded by the processor 4.

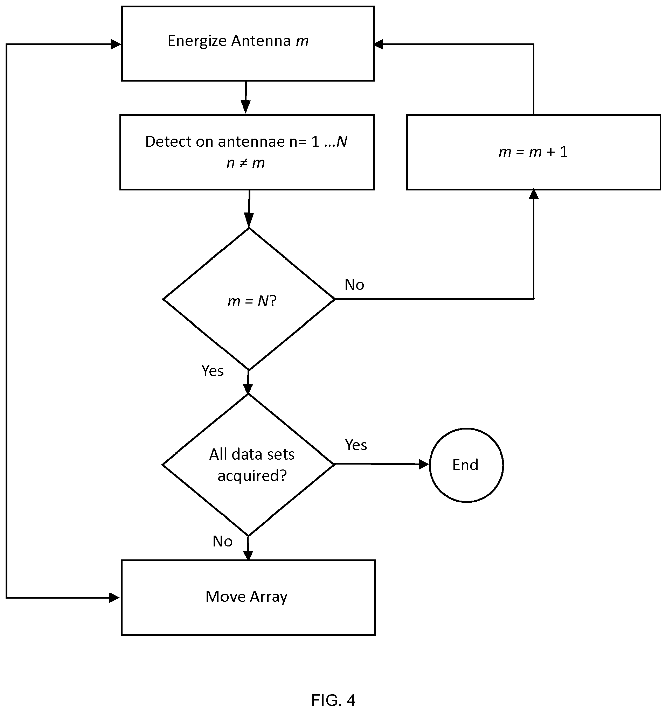

Figure 4 shows a flowchart of a data acquisition method. As shown, the switching matrix 20 connects an antenna *m* of the *N* antennae to the transmit path. All other antennae 16 (*n* = *1...N, n≠m*) are connected to the receive path and detect the transmitted signal (possibly in a time-sharing arrangement). If *m≠N*, the switching matrix 20 steps to the next antenna 16 (*m* = *m* + *1*) to be connected to the transmit path. This is repeated until all antennae 16 have been connected to the transmit path. The acquisition process may be repeated with the antenna array 6 translated (i.e. rotated about its rotational axis). This may allow fixed errors to be cancelled from the detected signals.

Referring again to Figure 3, the shell 18 receives a cup 30. The cup 30 has a complementary shape to the shell 18 such that if fits snugly within the shell 18.

The outside of the cup 30 and the inside of the shell 18 may have threaded portions to enable a threaded engagement between the cup 30 and the shell 18. The threaded engagement between the cup 30 and the shell 18 may be used to translate the antenna array 6 relative to the breast 36, as described previously.

A layer of coupling fluid (dielectric constant controlled fluid) may be inserted in the gap 31 between the shell 18 and the cup 30 so as to improve the coupling between the antennae 16 and the cup 30 in order to minimise signal loss and thus improve transmission of the microwave signal.

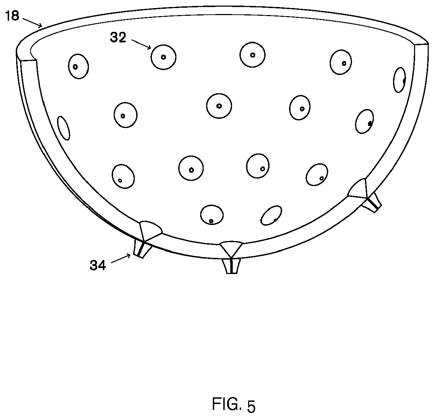

As shown in Figure 5, the shell 18 comprises a plurality of apertures 32 which are distributed across the surface of the shell 18. Each aperture 32 extends through the thickness of the shell 18 and tapers such that the aperture 32 is wider at the inner surface of the shell 18 than at the outer surface. The apertures 32 are therefore frustoconical. Each aperture 32 is provided with a sealing nozzle or gasket 34. The gaskets 34 are provided in order to prevent the coupling fluid from leaking through the apertures 32. The cup 30 also comprises a set of corresponding apertures in order to provide access from the exterior of the shell 18 through to the interior of the cup 30.

In use, a patient lies on the table 10 in a prone position such that their breast 36 sits in the cup 30. A layer of coupling fluid may also be provided in the gap 35 between the cup 30 and the breast 36 in order to improve coupling between the antennae 16 and the breast 36.

Although not shown, one or more inserts may be placed inside the cup 30 so as to enable a better fit between the internal surface of the cup 30 and the breast 36. For example, a plurality of such inserts may be provided, each having different shapes and sizes, to enable the system to be better adapted to breasts of different shapes and sizes. The inserts may be made from the same material as the cup (e.g. ceramic).

The antenna 16 connected to the transmit path illuminates the breast 36 with the microwave signal. The signal is scattered by the breast tissue and the scattered signal is received at each of the non-transmitting antennae 16 where it is detected and recorded. This process is repeated for each antenna 16, as described previously with reference to the data acquisition method shown in Figure 4.

The processor 4 may record the relative difference between the measured phase and amplitude of the transmitted signal as compared to the phase and amplitude of the scattered signal, recorded as a complex number (having real and imaginary parts).

The signal detected at each antenna 16 will generally comprise three components: a component arising from mutual coupling between the transmitting and receiving antennas 16; a component arising from radiation which reflects off the skin of the breast 36; and a component arising from radiation which reflects off structures within the breast (such as tumours). Tumours can generate identifiable reflections as they exhibit much higher dielectric properties than adipose tissues due to their significant water content. The mutual and skin reflection components may be removed or at least mitigated from the data set in order to improve the detectability of reflections resulting from the presence of tumours within the breast.

The acquired data set may be used by the processor 4 to construct an image of the internal structure of the breast 36. Data reconstruction may be performed using Phased Array (frequency domain), Delay and Sum (DAS - time domain) techniques or any other suitable technique. From this, the processor 4 is able to identify (possibly, with additional user input or confirmation) a region of interest (if present) in which a possible tumour or other pathology may exist. The presence of a tumour or other pathology may be confirmed by taking a biopsy from the region of interest.

Once the processor 4 (and any subsequent biopsy or other analysis) has identified the target, the delivery device may be used to deliver a marker to the site of the target (i.e. within or adjacent to the target). In particular, the processor 4 may guide the articulated arm 12 so as to locate the delivery needle 14 in the desired position and orientation for accessing the target. The articulated arm 12 may be a robotic arm such that it can manipulate the delivery needle 14 to the required position and orientation based on coordinates provided by the processor 4. Alternatively, the articulated arm 12 may be actuated by a user but provide feedback via appropriate sensors to the user or processor 4 of its current position so that it can be brought into the proper position and orientation. As a further alternative, the articulated arm 12 may be dispensed with and the delivery needle 14 itself provide feedback via sensors on its current position and orientation to the processor 4 or user.

As described previously, the shell 18 comprises a plurality of apertures 32 which provide access to the breast 36 for the delivery needle 14.

The processor 4 may determine the most appropriate aperture 32 for delivering the marker. In particular, the processor 4 may determine the closest aperture 32 to the target. As described previously, the apertures 32 are frustoconical such that the delivery needle 14 can be introduced through the aperture 32 at a range of angles.

With the delivery needle 14 guided to the appropriate position and orientation for accessing the region of interest, the delivery needle 14 can be inserted into the breast 36 and directed to the target. The antenna array 6 can be used to confirm that the needle is coincident with the target by again illuminating the breast 36 with microwave signals and detecting scattered signals from the delivery needle 14. Specifically, the processor 4 may confirm that the delivery needle 14 is appropriately positioned prior to final placement of the marker by updating the image. The image of the breast 36 (or only the relevant portion) may be updated as the delivery needle 14 passes into the breast 36 towards (and, when appropriate, into) the target.

During the guidance of the delivery needle 14, the processor 4 may adjust the signal processing performed such that it is optimised for speed and mapping a known object in space. The data acquisition and analysis performed by the processor during the guidance of the needle electrode 14 may be faster than during imaging for the purpose of initially identifying the region of interest.

With the needle electrode 14 positioned at or within the target region, the delivery device 8 may deliver a marker via the delivery needle 14.

Where the marker is placed immediately after a biopsy and before the result is known, the marker will typically be biodegradable. Hence, if the biopsy result indicates a non-cancerous lesion, the marker can be left in the body to biodegrade, otherwise it will be used for lesion localisation during surgery.

Alternatively, the biopsy result may be first assessed such that the patient only undergoes a second imaging procedure to relocate the lesion and deliver a marker if the result is cancerous.

The delivery needle may be manipulated to deliver additional markers under microwave image guidance, without having to create additional puncture wounds in the skin of tissue, if required.

In a subsequent surgical procedure for the removal of the lesion, the surgeon is guided to the lesion location using a marker detector.

The marker may be any known marker. For example, the marker may be a radiotracer or radioseed, such as those used in conventional ROLL or RSL procedures. With such markers, the marker detector would be a handheld gamma probe or the like. The marker may even form part of a wire guided localisation system.

Alternatively, the marker may be a magnetic or electromagnetic marker. For example, the marker could be ferromagnetic or ferrimagnetic or otherwise exhibit permanent magnetic properties. The stray field from a small cylindrical permanent magnet of dimensions radius 0.5mm, length 3mm, for example, could be detected outside the breast and be used to locate the lesion.

A detection system for such a magnetic marker may be based on an external reference framework of magnetic field sensors which is capable of providing a position and orientation readout of both the lesion marker and any surgical device. For example, an array of magnetic sensors (typically magnetorestive sensors or fluxgate magnetometers) may be used to measure the magnitude and direction of the stray field from the marker magnet. The inverse problem can then be solved to calculate the position and orientation of the marker (to within 1mm) and also any surgical device. Alternatively the position of the surgical device could be read by other means. Commercial products for magnet tracking are known in the art, such as those available from Matesy Gmbh, and so are not described in further detail here.

During the surgical procedure the surgeon may be presented with a visual and audio signal which indicates the relative positions of the surgical device and the marker so that the optimal surgical approach can be guided and carried out.

Alternatively, the magnetic marker may be detected using a handheld magnetic field probe which is manipulated by the surgeon to locate the lesion marker and guide the surgical device interactively.

Such probes typically incorporate a three-axis magnetic field sensor such as a fluxgate magnetometer in the probe tip. The information from such a sensor would be inadequate to uniquely determine the position of the marker, but could be used as a proximity probe with an audio feedback, in a similar fashion to a handheld gamma probe.

Other probe types based on AC susceptibility measurements could also be used.

While permanent magnetic materials generate a strong magnetic field they are also potentially toxic and non-biodegradable and so must be removed during surgery. As described previously, it may be preferable to utilise a biodegradable material, particularly where the marker is inserted at the same time as taking a biopsy. Biodegradable magnetic materials could therefore be used, such as magnetic nanoparticles based on magnetite, which are typically safe for clinical use. The magnetic nanoparticles may be encapsulated in a biodegradable matrix and inserted using a delivery needle, as described previously.

Alternatively, the marker may be an electromagnetic marker, and particularly a microwave marker, which is located using a radar probe.

Microwave markers will typically have one or more of the following characteristics:
- Enhanced overall microwave scattering amplitude over surrounding tissue
- Microwave scattering that is a strong function of frequency - the continuous wave radar is well suited to measure scattering as a function of frequency
- Scattering anisotropy - the hemispherical antenna array 6 is able to characterize scattering over a wide solid angle.

The marker should have a distinctive radar signature that can be characterised rapidly for guidance purposes. For example, the marker may include:
- Radio frequency identification tags
- Microwave metamaterials
- Ferromagnetically or ferrimagnetically resonant materials within the UWB band, such as ferrites.

With microwave markers, the microwave radar probe does not need to form an image of the body part, but simply indicates to the surgeon the position and orientation of the marker with respect to the probe.

Delivery needles are typically made of steel which generally provides a strong microwave scattering signal and can therefore be reliably located. However, the delivery needle may bend as it passes through tissue, and/or the scattering signal may be modified by surrounding tissue. To allow for either of these more complex cases, the delivery needle may be modified so that it is more easily identifiable. In particular, the delivery needle may be provided with a guidance marker which is more easily discriminated by microwave radar in order to determine needle tip location and overall orientation more accurately.

The guidance marker may be a passive marker, as described previously, which scatters the microwave signals generated by the antennae 16. The guidance marker may be formed by the localization marker which is implanted at the site of the target once correctly placed.

In accordance with the invention, the delivery needle 14 itself radiates the microwave signal, rather than scattering signals generated by the antennae 16. For example, the delivery needle 14 has an integrated coaxial feed line which supplies the signal to the tip of the needle. Alternatively, the delivery needle 14 may comprise an integrated microwave source.

The antennae 16 of the antenna array 6 therefore all (or a subset of the antennae 16) act to receive the signal emitted by the delivery needle 14. Using the delivery needle 14 as the signal source avoids having to sequentially connect each of the antennae 16 to the transmit path and thus allows the scan time to be drastically reduced, potentially allowing real time operation.

Although the delivery needle 14 has been described as being automatically guided to the target, it may instead be manually guided, with its location being tracked inside the body part and displayed on a model of the body part along with the target's location so that it can be guided towards the target. A vector which extends from the end of the delivery needle may be projected onto the image to show the trajectory of the needle and to help the clinician guide the needle towards the target. The image may be repeatedly updated as the needle moves towards the target.

Although the shell 18 has been described as having a plurality of frustoconical apertures 32 for providing access to the breast, other arrangements may be used. In particular, the shell 18 may have a plurality of circumferential slots. The angular spacing of the slots corresponds to the available angular rotation of the antenna array 6. Each slot, under rotation, gives access to a volume of tissue which typically overlaps to some degree with adjacent slots in order to offer some options in needle placement.

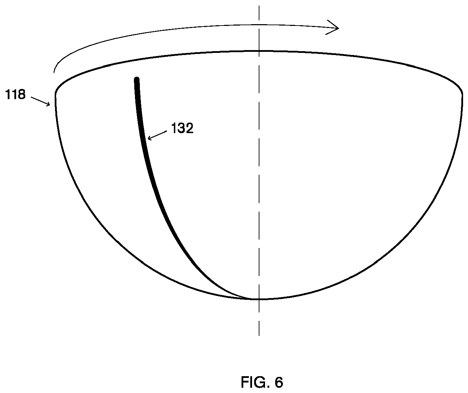

In particular, Figure 6 shows an alternative embodiment of a shell 118 which may be used where the antenna array 6 is able to rotate through 180°. The shell 118 comprises a single slot 132 which passes through the axis of rotation from each side of the shell 118 (or two diametrically opposed slots with a discontinuity at the axis of rotation). The shell 118 is rotatable through 180° such that the slot 132 is able to allow the delivery needle 14 to access the entire breast 36.

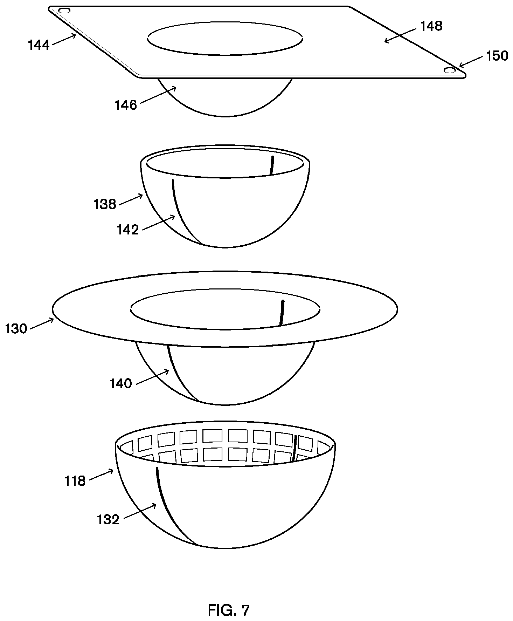

As shown in Figure 7, the shell 118 is used with a cup 130 and, if required, an insert 138. The cup 130 has a complementary shape to the shell 118 such that if fits snugly within the shell 118 and, in turn, the insert 138 has a complementary shape to the cup 130 such that if fits snugly within the cup 130.

The cup 130 is provided with a slot 140 which corresponds to the slot 132 of the shell 118. Similarly, the insert 138 is provided with a slot 142 which corresponds to the slot 132 of the shell 118. The cup 130 and, if used, the insert 138 are disposed within the shell 118 such that the slots 132, 140, 142 of the shell 118, cup 130 and insert 138 are all aligned with one another. The slots 132, 140, 142 therefore provide access to the breast 36 from the exterior of the shell 118. The cup 130 and insert 138 are connected to the shell 118 so as to prevent relative rotation and thus to maintain the alignment of the slots 132, 140, 142. In particular, the cup and insert may be provided with locating tabs which prevent rotation relative to the shell 118.

As described with respect to the previous embodiment, a layer of coupling fluid may be inserted in the gap between the shell 118 and the cup 130 and also in the gap between the cup 130 and the insert 138. The slots 132, 140, 142 may be fitted with a gasket to prevent the escape of coupling fluid.

To allow the shell 118 to rotate relative to the breast 36, a disposable tray 144 is used. The tray 144 is made from a biocompatible polymer, such as PEEK, HIPS, PET. The tray comprises a central section 146 which is curved to conform to the underlying cup 130 or insert 138. In particular, the central section 146 may be part or hemi-spherical. Disposable trays may be provided in different sizes that correspond to the internal dimensions of each available insert (and cup) respectively so that a low-compliance fit to the underlying surface is achieved. A planar rim 148 extends from the central section 146. The material thickness over the central section 146 may be less than that of the planar rim 148. The planar rim 148 may therefore provide structural rigidity to the disposable tray 144, while the thin central section 146 ensures that the dielectric properties of the matching interface between the cup/inserts and patient is not disturbed.

The disposable tray 144 is held stationary against the breast 36 while the shell 118, cup 130 and insert 138 are rotated to properly align the slots 132, 140, 142 with the desired location over the breast 36 from which to insert the marker. The disposable tray 144 may be provided with location tabs 150 or the like which engage with complementary features on a stationary housing (not shown) of the antenna array 6. The disposable tray 144 is therefore fixed in position and prevented from rotating with the shell 118 and the intermediate component(s). The disposable tray 144 provides little resistance to the rotation of the shell 118 and so the patient does not feel the shell 118 being moved into position.

It will be appreciated that the disposable tray 144 lies over the aligned slots 132, 140, 142 and so obstructs the delivery needle 14. However, the material of the disposable tray 144 is suitably thin than it can be punctured by the needle. The needle punctures the tray 144 and breast tissue at a sufficiently high speed and force to minimise the breast tissue shrinking away from the surface of the tray 144. This therefore ensures that the needle is guided accurately to the target. Further, at least over the central section 146, the material of the disposable tray 144 may have low-shear characteristics such that when it is punctured by the delivery needle, the fracturing characteristic of the polymer can be controlled.

A drainage arrangement may be included to ensure that any coupling fluid that flows out of the slots in the array is channelled away from the main electro-mechanical components of the system.

The system described herein is capable of both providing a clinical breast microwave image showing a suspicious lesion and also guiding delivery of a marker to the lesion so as to allow the lesion to be easily located during a subsequent surgical procedure.

As the guidance of the marker is performed by the same system which identifies the target, the patient may potentially remain in position throughout the procedure such that the breast retains registration throughout.

The system provides significant benefits over previously known systems and techniques. In particular, the system provides precise positioning of the delivery needle with respect to the acquired image, so that the clinician has a high degree of confidence that the proposed trajectory of the needle will intersect the target. This reduces the number of needle insertions/withdrawals and the risk of seeding malignant cells along multiple needle tracks.

As described previously, the target may be identified by performing a biopsy of a region of interest. The guidance of the biopsy may be achieved in a similar manner to that used for delivering the marker to the target. In fact, the delivery needle may also function as a biopsy needle or be replaced by a biopsy needle during this procedure. Alternatively, a separate biopsy probe may be provided as part of the system.

The invention may utilise any known type of delivery device for delivering a marker to a target.

Although the system has been described with reference to the imaging of a breast, it will be appreciated that it may be adapted for other areas of the body.

It will be appreciated that the processor 4 need not display, or indeed, generate, an image of the internal structure of the breast in order to identify a region of interest. The region of interest may instead be determined based on the raw data.

In other arrangements, the cup of the antenna array may be dispensed with or integrated into the shell in which the antennae are located.

The system may employ multiple transmit and receive paths, such that data can be recorded from multiple antennas simultaneously, and may even comprise a number of transmit and receive paths corresponding to the number of antennas, such that data can be recorded from all antennas simultaneously. In an alternative topology, the switching matrix could be removed thus allowing each antenna to be connected to a transmitting/receiving device.

Alternatively, more than one antenna may be operated at a time in a frequency multiplexed operation. Multiplexed operation may have significant advantages where high speed tracking of the needle is required.

To avoid unnecessary duplication of effort and repetition of text in the specification, certain features are described in relation to only one or several aspects or embodiments of the invention. However, it is to be understood that, where it is technically possible, features described in relation to any aspect or embodiment of the invention may also be used with any other aspect or embodiment of the invention.

The invention is not limited to the embodiments described herein, and may be modified or adapted without departing from the scope of the present invention.

## Claims

1. A medical imaging system (2) comprising:
a microwave antenna array (6) comprising a transmitting antenna (16) and a plurality of receiving antennae (16), wherein the transmitting antenna is configured to transmit microwave signals so as to illuminate a body part (36) of a patient and the receiving antennae are configured to receive the microwave signals following scattering within the body part (36);
a processor (4) configured to process the scattered microwave signals and generate an output indicative of the internal structure of the body part (36) to identify a target within the body part (36); and
a delivery device (8) comprising a delivery needle (14) for implanting a marker at the site of the target, the delivery device (8) being movable relative to the microwave antenna array (6); **characterised in that**:
the delivery needle (14) has an integrated coaxial feed line for supplying microwave signals to the tip of the needle (14), such that the delivery needle (14) itself is configured to radiate microwave signals;
the receiving antennae are further configured to receive microwave signals radiated by the delivery needle (14); and
the processor (4) is further configured to use the received microwave signals radiated by the delivery needle (14) to monitor a position of the delivery needle (14) as it is guided to the target within the body part (36).

2. A medical imaging system as claimed in claim 1, wherein the processor (4) is further configured to guide the delivery needle (14) to the target within the body part (36).

3. A medical imaging system (2) as claimed in claim 1 or 2, wherein the microwave antenna array (6) is formed on a substrate, wherein the substrate comprises one or more openings configured to receive the delivery needle (14) to provide access to the body part.

4. A medical imaging system (2) as claimed in claim 3, wherein the openings are conical.

5. A medical imaging system (2) as claimed in claim 3 or 4, wherein the openings are provided with sealing gaskets.

6. A medical imaging system (2) as claimed in any of claims 3 to 5, wherein the openings comprise one or more slots.

7. A medical imaging system (2) as claimed in any of claims 2 to 5, wherein the substrate comprises a plurality of said openings and wherein the processor (4) is configured to select one of the plurality of openings for introducing the delivery needle (14).

8. A medical imaging system (2) as claimed in any preceding claim, wherein the delivery device is mounted on an articulated arm configured to maneuver the delivery device relative to the antenna array (6).

9. A medical imaging system (2) as claimed in claim 8, wherein the articulated arm is a robotic arm.

10. A medical imaging system (2) as claimed in any preceding claim, wherein the marker is a microwave marker which is provided at the tip of the delivery needle (14) and wherein the processor (4) is configured to monitor the position of the delivery needle (14) using the marker as it is guided to the target within the body part before being implanted.

11. A medical imaging system (2) as claimed in any preceding claim, wherein the processor (4) is configured to perform a first data acquisition and analysis operation when identifying the target and to perform a second data acquisition and analysis operation when guiding the delivery needle (14), the second data acquisition and analysis operation being faster than the first data acquisition and analysis operation.

12. A medical imaging system (2) as claimed in any preceding claim, further comprising a marker detector, wherein the marker detector is configured to detect the marker after it is implanted at the site of the target in order to locate the target during a surgical procedure.

13. A medical imaging system (2) as claimed in any preceding claim, wherein the marker is a magnetic marker.

14. A medical imaging system (2) as claimed in any preceding claim, wherein the marker is an electromagnetic marker.

15. A medical imaging system (2) as claimed in any preceding claim, wherein the marker is biodegradable.

16. A medical imaging system (2) as claimed in any preceding claim, wherein the delivery needle (14) is configured to extract a biopsy from the target.

## Patentansprüche

1. Medizinisches Bildgebungssystem (2), umfassend:
eine Mikrowellenantennenanordnung (6), umfassend eine Übertragungsantenne (16) und eine Vielzahl von Empfangsantennen (16), wobei die Übertragungsantenne zum Übertragen von Mikrowellensignalen zum Beleuchten eines Körperteils (36) eines Patienten ausgelegt ist und die Empfangsantennen zum Empfangen der Mikrowellensignale nach der Streuung im Körperteil (36) ausgelegt sind;
einen Prozessor (4), der zum Verarbeiten der gestreuten Mikrowellensignale und zum Erzeugen einer Ausgabe, die auf die interne Struktur des Körperteils (36) zur Identifizierung eines Ziels innerhalb des Körperteils (36) hinweist, ausgelegt ist; und
eine Abgabevorrichtung (8), die eine Abgabenadel (14) zur Implantation eines Markers an der Stelle des Ziels umfasst, wobei die Abgabevorrichtung (8) bezüglich der Mikrowellenantennenanordnung (6) beweglich ist; **dadurch gekennzeichnet, dass**:
die Abgabenadel (14) eine integrierte koaxiale Zuführungsleitung zum Zuführen von Mikrowellensignalen zur Spitze der Nadel (14) aufweist, so dass die Abgabenadel (14) selbst zum Abstrahlen von Mikrowellensignalen ausgelegt ist;
die Empfangsantennen ferner zum Empfangen von Mikrowellensignalen ausgelegt sind, die von der Abgabenadel (14) abgestrahlt wurden; und
der Prozessor (4) ferner dazu ausgelegt ist, die von der Abgabenadel (14) abgestrahlten empfangenen Mikrowellensignale zur Überwachung einer Position der Abgabenadel (14), während diese zum Ziel innerhalb des Körperteils (36) geführt wird, zu verwenden.

2. Medizinisches Bildgebungssystem nach Anspruch 1, wobei der Prozessor (4) ferner zum Führen der Abgabenadel (14) zum Ziel innerhalb des Körperteils (36) ausgelegt ist.

3. Medizinisches Bildgebungssystem (2) nach Anspruch 1 oder 2, wobei die Mikrowellenantennenanordnung (6) auf einem Substrat ausgebildet ist, wobei das Substrat eine oder mehrere Öffnungen umfasst, die zur Aufnahme der Abgabenadel (14) ausgelegt sind, um Zugang zum Körperteil bereitzustellen.

4. Medizinisches Bildgebungssystem (2) nach Anspruch 3, wobei die Öffnungen konisch sind.

5. Medizinisches Bildgebungssystem (2) nach Anspruch 3 oder 4, wobei die Öffnungen Dichtungen aufweisen.

6. Medizinisches Bildgebungssystem (2) nach einem der Ansprüche 3 bis 5, wobei die Öffnungen einen oder mehrere Schlitze umfassen.

7. Medizinisches Bildgebungssystem (2) nach einem der Ansprüche 2 bis 5, wobei das Substrat eine Vielzahl dieser Öffnungen umfasst und wobei der Prozessor (4) dazu ausgelegt ist, eine der Vielzahl von Öffnungen zur Einführung der Abgabenadel (14) auszuwählen.

8. Medizinisches Bildgebungssystem (2) nach einem der vorhergehenden Ansprüche, wobei die Abgabevorrichtung auf einem gelenkigen Arm angebracht ist, der zum Manövrieren der Abgabevorrichtung bezüglich der Antennenanordnung (6) ausgelegt ist.

9. Medizinisches Bildgebungssystem (2) nach Anspruch 8, wobei der gelenkige Arm ein Roboterarm ist.

10. Medizinisches Bildgebungssystem (2) nach einem der vorhergehenden Ansprüche, wobei der Marker ein Mikrowellenmarker ist, der an der Spitze der Abgabenadel (14) vorgesehen ist und wobei der Prozessor (4) dazu ausgelegt ist, die Position der Abgabenadel (14) unter Verwendung des Markers während der Führung zum Ziel innerhalb des Körperteils vor der Implantation zu überwachen.

11. Medizinisches Bildgebungssystem (2) nach einem der vorhergehenden Ansprüche, wobei der Prozessor (4) zur Durchführung eines ersten Datenerfassungs- und Analysevorgangs bei der Identifizierung des Ziels und zur Durchführung eines zweiten Datenerfassungs- und Analysevorgangs bei der Führung der Abgabenadel (14) ausgelegt ist, wobei der zweite Datenerfassungs- und Analysevorgang schneller als der erste Datenerfassungs- und Analysevorgang ist.

12. Medizinisches Bildgebungssystem (2) nach einem der vorhergehenden Ansprüche, ferner umfassend einen Markerdetektor, wobei der Markerdetektor zum Nachweisen des Markers nach dessen Implantation an der Stelle des Ziels zur Lokalisierung des Ziels während eines chirurgischen Verfahrens ausgelegt ist.

13. Medizinisches Bildgebungssystem (2) nach einem der vorhergehenden Ansprüche, wobei der Marker ein magnetischer Marker ist.

14. Medizinisches Bildgebungssystem (2) nach einem der vorhergehenden Ansprüche, wobei der Marker ein elektromagnetischer Marker ist.

15. Medizinisches Bildgebungssystem (2) nach einem der vorhergehenden Ansprüche, wobei der Marker biologisch abbaubar ist.

16. Medizinisches Bildgebungssystem (2) nach einem der vorhergehenden Ansprüche, wobei die Abgabenadel (14) zum Entnehmen einer Biopsie aus dem Ziel ausgelegt ist.

## Revendications

1. Système d'imagerie médicale (2) comprenant :
un réseau (6) d'antennes micro-ondes comprenant une antenne émettrice (16) et une pluralité d'antennes réceptrices (16), dans lequel l'antenne émettrice est conçue pour émettre des signaux micro-ondes de façon à éclairer une partie corporelle (36) d'un patient et les antennes réceptrices sont conçues pour recevoir les signaux micro-ondes après diffusion à l'intérieur de la partie corporelle (36) ;
un processeur (4) configuré pour traiter les signaux micro-ondes diffusés et pour générer une sortie représentative de la structure interne de la partie corporelle (36) dans le but d'identifier une cible à l'intérieur de la partie corporelle (36) ; et
un dispositif de pose en place (8) comprenant une aiguille de pose en place (14) pour implanter un marqueur au niveau du site de la cible, le dispositif de pose en place (8) étant mobile par rapport au réseau (6) d'antennes micro-ondes ; **caractérisé en ce que** :
l'aiguille de pose en place (14) possède une ligne d'alimentation coaxiale intégrée pour fournir des signaux micro-ondes à la pointe de l'aiguille (14), de sorte que l'aiguille de pose en place (14) elle-même soit conçue pour émettre des signaux micro-ondes ;
les antennes réceptrices sont en outre conçues pour recevoir des signaux micro-ondes émis par l'aiguille de pose en place (14) ; et
le processeur (4) est en outre conçu pour utiliser les signaux micro-ondes reçus émis par l'aiguille de pose en place (14) dans le but de surveiller une position de l'aiguille de pose en place (14) à mesure qu'elle est guidée vers la cible à l'intérieur de la partie corporelle (36).

2. Système d'imagerie médicale selon la revendication 1, dans lequel le processeur (4) est en outre configuré pour guider l'aiguille de pose en place (14) vers la cible à l'intérieur de la partie corporelle (36).

3. Système d'imagerie médicale (2) selon la revendication 1 ou 2, dans lequel le réseau (6) d'antennes micro-ondes est formé sur un substrat, dans lequel le substrat comprend une ou plusieurs ouvertures conçues pour recevoir l'aiguille de pose en place (14) dans le but de permettre l'accès à la partie corporelle.

4. Système d'imagerie médicale (2) selon la revendication 3, dans lequel les ouvertures sont coniques.

5. Système d'imagerie médicale (2) selon la revendication 3 ou 4, dans lequel les ouvertures sont pourvues de joints d'étanchéité.

6. Système d'imagerie médicale (2) selon l'une quelconque des revendications 3 à 5, dans lequel les ouvertures comprennent une ou plusieurs fentes.

7. Système d'imagerie médicale (2) selon l'une quelconque des revendications 2 à 5, dans lequel le substrat comprend une pluralité desdites ouvertures et dans lequel le processeur (4) est conçu pour sélectionner une ouverture de la pluralité d'ouvertures pour introduire l'aiguille de pose en place (14).

8. Système d'imagerie médicale (2) selon l'une quelconque des revendications précédentes, dans lequel le dispositif de pose en place est monté sur un bras articulé conçu pour manœuvrer le dispositif de pose en place par rapport au réseau (6) d'antennes .

9. Système d'imagerie médicale (2) selon la revendication 8, dans lequel le bras articulé est un bras robotique.

10. Système d'imagerie médicale (2) selon l'une quelconque des revendications précédentes, dans lequel le marqueur est un marqueur micro-ondes qui est prévu à la pointe de l'aiguille de pose en place (14) et dans lequel le processeur (4) est configuré pour surveiller la position de l'aiguille de pose en place (14) à l'aide du marqueur à mesure qu'il est guidé vers la cible à l'intérieur de la partie corporelle pour être implanté.

11. Système d'imagerie médicale (2) selon l'une quelconque des revendications précédentes, dans lequel le processeur (4) est configuré pour réaliser une première opération d'acquisition et d'analyse de données lors de l'identification de la cible et pour réaliser une seconde opération d'acquisition et d'analyse de données lors du guidage de l'aiguille de pose en place (14), la seconde opération d'acquisition et d'analyse de données étant plus rapide que la première opération d'acquisition et d'analyse de données.

12. Système d'imagerie médicale (2) selon l'une quelconque des revendications précédentes, comprenant en outre un détecteur de marqueur, dans lequel le détecteur de marqueur est conçu pour détecter le marqueur après qu'il a été implanté au niveau du site de la cible dans le but de localiser la cible pendant une intervention chirurgicale.

13. Système d'imagerie médicale (2) selon l'une quelconque des revendications précédentes, dans lequel le marqueur est un marqueur magnétique.

14. Système d'imagerie médicale (2) selon l'une quelconque des revendications précédentes, dans lequel le marqueur est un marqueur électromagnétique.

15. Système d'imagerie médicale (2) selon l'une quelconque des revendications précédentes, dans lequel le marqueur est biodégradable.

16. Système d'imagerie médicale (2) selon l'une quelconque des revendications précédentes, dans lequel l'aiguille de pose en place (14) est conçue pour extraire une biopsie de la cible.
